# EUROPÄISCHE PATENTSCHRIFT

(11) **EP 0 732 894 B1**
(45) Veröffentlichungstag und Bekanntmachung des Hinweises auf die Patenterteilung: **21.01.1998**
(21) Anmeldenummer: 94930175.8
(22) Anmeldetag: 15.10.1994
(51) Int. Cl.: A61B 17/28

(54) **INSTRUMENT FÜR CHIRURGISCHE ZWECKE**
INSTRUMENT FOR SURGICAL PURPOSES
INSTRUMENT A UTILISATION CHIRURGICALE

(30) Priorität: 08.12.1993 DE 4341734
(43) Veröffentlichungstag der Anmeldung: 25.09.1996
(73) Patentinhaber: AESCULAP AG, 78532 Tuttlingen (DE)
(72) Erfinder: GRIMM, Holger, D-78652 Deisslingen (DE); HEBESTREIT, Klaus, D-78532 Tuttlingen (DE); MORALES, Pedro, D-78532 Tuttlingen (DE)
(74) Vertreter: Böhme, Ulrich Hoeger, Stellrecht & Partner
(86) Internationale Anmeldenummer: EP9403400
(87) Internationale Veröffentlichungsnummer: WO9515722

(56) Entgegenhaltungen:
- DE-U- 9 202 132
- DE-U- 9 307 622
- US-A- 4 369 788
- US-A- 4 712 545

## Beschreibung

Die Erfindung betrifft ein Instrument für chirurgische Zwecke mit zwei relativ zueinander verschiebbaren Arbeitsteilen und mit zwei relativ zueinander verschwenkbaren Griffbranchen, von denen eine mit dem einen und die andere mit dem anderen Arbeitsteil derart in Verbindung stehen, daß beim Schließen beziehungsweise Öffnen der Griffbranchen die beiden Arbeitsteile gegeneinander verschoben werden, wobei eine der beiden Griffbranchen eine Lagerwelle zur schwenkbaren Lagerung der anderen Griffbranche trägt, die Bereiche mit unterschiedlichem Durchmesser aufweist und derart axial verschieblich ist, daß im Bereich der anderen Griffbranche wahlweise ein Bereich größeren oder ein Bereich kleineren Durchmessers wirksam ist, wobei die andere Griffbranche einen Schlitz für die Lagerwelle mit einem schmaleren Mittelabschnitt und einem erweiterten Lagerabschnitt an einem Ende aufweist, dessen Breite im schmaleren Mittelabschnitt zwischen dem Durchmesser der beiden Bereiche der Lagerwelle liegt und in dem kreisförmig erweiterten Lagerabschnitt einen Durchmesser aufweist, der dem größeren Durchmesser der Lagerwelle entspricht, und wobei eine der beiden Griffbranchen mit einem Mitnehmer gelenkig an dem einen Arbeitsteil angreift, wenn die Lagerwelle den erweiterten Lagerabschnitt durchsetzt.

Instrumente dieser Art werden im chirurgischen Bereich vielfältig eingesetzt, beispielsweise in Form von sogenannten Rohrschaftinstrumenten oder von Biopsiezangen etc. Um diese Instrumente zu Reinigungszwecken und zum Austausch von Teilen zerlegen zu können, ist es notwendig, die Verbindung zwischen den Arbeitsteilen einerseits und den Griffbranchen andererseits zu lösen. Dies ist insbesondere bei sehr kleinen Instrumenten zum Teil recht aufwendig, da für die Übertragung der hohen Kräfte zuverlässige Verbindungen notwendig sind, die nicht in einfacher Weise gelöst werden können.

Es sind bereits derartige Instrumente bekannt, bei welchen eine Lösung der Wirkverbindung zwischen einer Griffbranche und einem verschiebbaren Arbeitsteil dadurch erfolgt, daß die Griffbranche in ihrer Lagerung an der anderen Griffbranche längsverschieblich ausgeführt ist, so daß bei der Längsverschiebung der Eingriff mit dem Arbeitsteil verloren geht (DE-U-93 07 622). Allerdings ist die Ausführung dort so gewählt, daß bei der Lösung der Wirkverbindung zwischen einer Griffbranche und einem bewegten Arbeitsteil zwangsläufig die Griffbranchen vollständig voneinander gelöst werden, das heißt, es wird das gesamte Instrument zerlegt.

Es ist Aufgabe der Erfindung, ein gattungsgemäßes Instrument so weiterzubilden, daß eine Lösung der Wirkverbindung zwischen Griffbranche und Arbeitsteil möglich ist, ohne daß eine vollständige Zerlegung des Instruments im Bereich der Griffbranchen notwendig wird.

Diese Aufgabe wird bei einem Instrument der eingangs beschriebenen Art erfindungsgemäß dadurch gelöst, daß am anderen Ende des Mittelabschnitts ein anderer erweiterter Lagerabschnitt mit einem ebenso erweiterten Durchmesser angeordnet ist und daß der Mitnehmer das Arbeitsteil freigibt, wenn die Lagerwelle diesen anderen erweiterten Lagerabschnitt durchsetzt.

Es werden also zwei Lagerpositionen vorgesehen, in denen die Griffbranchen gelenkig miteinander verbunden sind, und zwischen diesen zwei Lagerpositionen können die beiden Griffbranchen gegeneinander verschoben werden, wobei in einer Lagerposition ein Eingriff mit dem Arbeitsteil vorliegt, in der anderen Lagerposition nicht. Bei dieser Konstruktion ist sichergestellt, daß die Griffbranchen immer gelenkig miteinander verbunden bleiben, auch wenn die Wirkverbindung mit dem Arbeitsteil gelöst wird.

Vorteilhaft ist es, wenn die Welle unter Federbelastung in eine Position verschoben wird, in der der Bereich mit größerem Durchmesser an der auf der Welle gelagerten Griffbranche wirksam ist. Damit wird gewährleistet, daß die Welle normalerweise in eine der beiden Lagerendpositionen eingeschoben ist, so daß die Griffbranchen einwandfrei geführt aneinander gelagert sind. Zum Verschieben zwischen den beiden Positionen und zum Durchtritt durch-den engeren Mittelabschnitt muß die Welle gegen die Federbelastung verschoben werden.

Bei einer bevorzugten Ausführungsform ist vorgesehen, daß die Welle einen sich an der Außenseite einer Griffbranche abstützenden Kopf aus elastisch verformbarem Material aufweist, der sich unter dauerhafter Abstützung an der Griffbranche über den gesamten axialen Verschiebeweg der Welle elastisch verformen läßt. Es wird dadurch möglich, auf getrennte Federelemente zu verzichten, beispielsweise sind bei dieser Ausführungsform keine Schraubenfedern mehr notwendig, da die rückstellende Federbelastung durch die elastische Verformung des Kopfes hervorgerufen wird.

Insbesondere ist es günstig, wenn der Kopf im unverformten Zustand die Form einer etwa kugeligen Halbschale aufweist, die sich mit ihrem Rand an der Griffbranche abstützt und die in ihrem zentralen Teil mit der Welle verbunden ist. Dadurch ist ein relativ großer Hubweg möglich, ohne daß der Kopf seitlich allzu große Abmessungen benötigt.

Bei einer weiteren bevorzugten Ausführungsform ist dabei vorgesehen, daß die Welle eine stufige Metallhülse umfaßt, in die ein mit dem Kopf verbundener Schaft eingesteckt ist, insbesondere kann von der gegenüberliegenden Seite des Schaftes ein die Metallhülse stirnseitig überfangendes Gegenstück in diese eingeführt sein, welches mit dem Schaft verbunden ist. Besonders vorteilhaft ist dabei eine Verbindung zwischen Schaft und Gegenstück durch eine Rastverbindung.

Bei einer bevorzugten Ausführungsform, die vorzugsweise bei Rohrschaftinstrumenten eingesetzt wird, ist vorgesehen, daß das bewegte Arbeitsteil ein kugeliges Ende aufweist, das über einen Steg mit kleineren Abmessungen mit dem Arbeitsteil in Verbindung steht.

Dabei kann vorgesehen sein, daß der Mitnehmer eine im wesentlichen parallel zum Schlitz verlaufende Sacklochbohrung in der Griffbranche ist, die über einen seitlichen Längsschlitz offen ist, dessen Breite kleiner ist als der Durchmesser des kugeligen Endes und größer als die Breite des Steges.

Vorzugsweise ist der Schlitz im wesentlichen senkrecht zur Verschieberichtung des Arbeitsteils angeordnet.

Die nachfolgende Beschreibung bevorzugter Ausführungsformen der Erfindung dient im Zusammenhang mit der Zeichnung der näheren Erläuterung. Es zeigen:

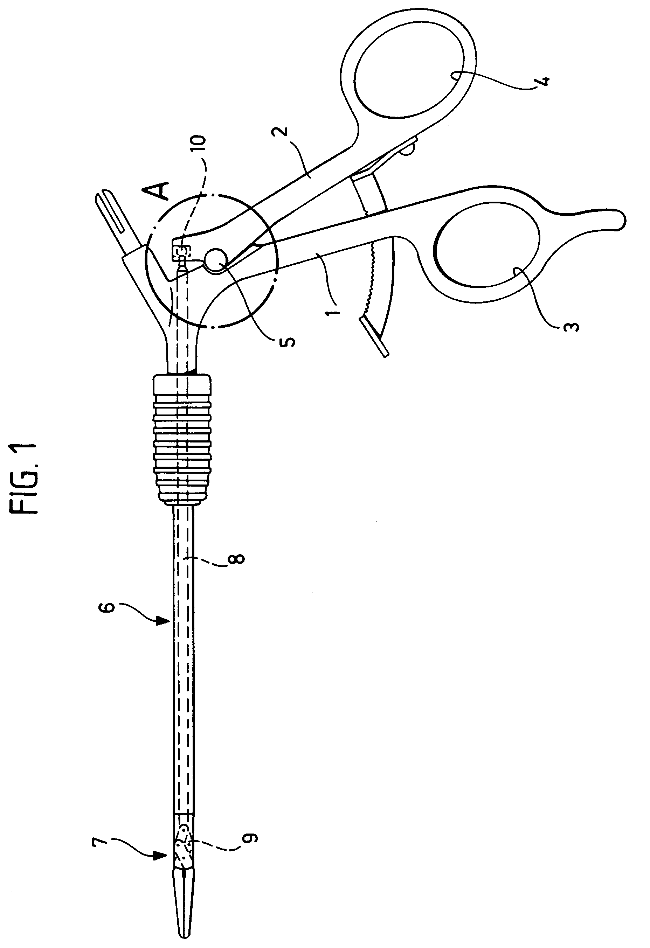

- Figur 1:: eine Seitenansicht eines Rohrschaftinstruments;
- Figur 2:: eine vergrößerte, teilweise aufgebrochene Darstellung des Bereiches A in Figur 1 bei Eingriff der Griffbranche mit dem Arbeitsteil;

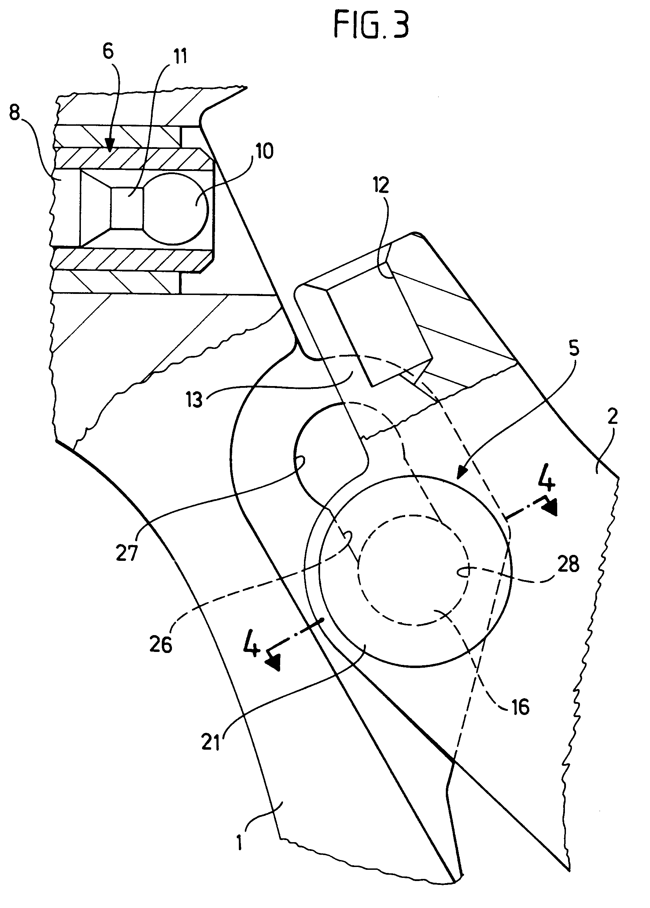

- Figur 3:: eine Ansicht ähnlich Figur 2 bei vom Arbeitsteil gelöster Griffbranche;
- Figur 4:: eine Schnittansicht längs Linie 4-4 in Figur 3 mit nicht eingeschobener Welle und
- Figur 5:: eine Ansicht ähnlich Figur 4 mit in Richtung des Pfeiles eingeschobener Welle.

Das in der Zeichnung dargestellte Rohrschaftinstrument umfaßt zwei Griffbranchen 1 und 2 mit Fingeröffnungen 3 beziehungsweise 4, die an einer Lagerstelle 5 schwenkbar miteinander verbunden sind. Eine der beiden Griffbranchen 1 trägt einen rohrförmigen Schaft 6, an dessen freiem Ende ein Werkzeug 7 gehalten ist. Dieses Werkzeug 7 ist gelenkig mit einem stangenförmigen Arbeitsteil 8 verbunden, das längsverschieblich im Schaft 6 angeordnet ist und an dessen Rückseite aus diesem hervorsteht. Dort ist das Arbeitsteil 8 gelenkig mit der anderen Griffbranche 2 verbunden, so daß es beim Öffnen und Schließen der Griffbranchen 1 und 2 im Schaft 6 hin- und hergeschoben wird. Dabei betätigt das Arbeitsteil 8 über Getriebeteile 9 das Werkzeug 7. Dieses Werkzeug 7 kann eine schneidende Funktion haben oder eine Zange sein, es sind auch andere Werkzeuge möglich.

Die gelenkige Verbindung zwischen dem aus dem Schaft 6 hervorstehenden Ende des Arbeitsteils 8 einerseits und der Griffbranche 2 andererseits erfolgt über ein kugeliges Ende 10 am Arbeitsteil 8, das über einen Steg 11 mit dem Arbeitsteil 8 verbunden ist. Dieser Steg 11 weist geringere Querabmessungen auf als das kugelige Ende 10, das heißt, das Arbeitsteil 8 ist im Bereich des Stegs 11 eingeschnürt und gegenüber seinem sonstigen Außenumfang zurückgesetzt.

In der Griffbranche 2 befindet sich eine Sacklochbohrung 12 mit einer seitlichen, längsschlitzförmigen Öffnung 13. Die Sacklochbohrung 12 nimmt das kugelige Ende 10 des Arbeitsteils 8 auf, die schlitzförmige Öffnung 13 den Steg 11. Die Öffnung 13 ist dabei weniger breit als das kugelige Ende 10 des Arbeitsteils 8, so daß bei einer Verschwenkung der Griffbranche 2 das in der Sacklochbohrung 12 umfangene kugelige Ende 10 mitgenommen wird. Der Innendurchmesser der Sacklochbohrung 12 entspricht dabei vorzugsweise dem Durchmesser des kugeligen Endes 10, so daß sich auch Druckbewegungen unmittelbar auf das Arbeitsteil 8 übertragen.

Im Bereich der Lagerstelle 5 trägt die Griffbranche 2, die im Bereich der Lagerstelle zweilagig ausgebildet ist und die Griffbranche 1 zwischen sich aufnimmt, zwei miteinander ausgerichteten Öffnungen 14 und 15 eine Lagerwelle 16. Diese wird gebildet von einer sich stufig erweiternden Metallhülse 17, in die einseitig ein mit einem Schaft 18 versehenes Kopfteil 19 eingesteckt ist. Von der anderen Seite ist ein Gegenstück 20 in die Metallhülse 17 hineingesteckt, welches die Stirnseite der Metallhülse 17 übergreift und welches mit dem Schaft 18 durch eine in der Zeichnung nicht gesondert dargestellte Rastverbindung verbunden ist. Das Kopfteil 19 weist einen Kopf 21 in Form einer kugeligen Halbschale auf, der sich mit seinem Rand 22 an der Außenseite der Griffbranche 2 abstützt und der in seinem zentralen Bereich mit dem Schaft 18 verbunden ist. Das Kopfteil 19 besteht aus einem elastisch verformbaren Material, vorzugsweise aus einem elastomeren Kunststoff. Auch das Gegenstück 20 kann aus Kunststoff bestehen.

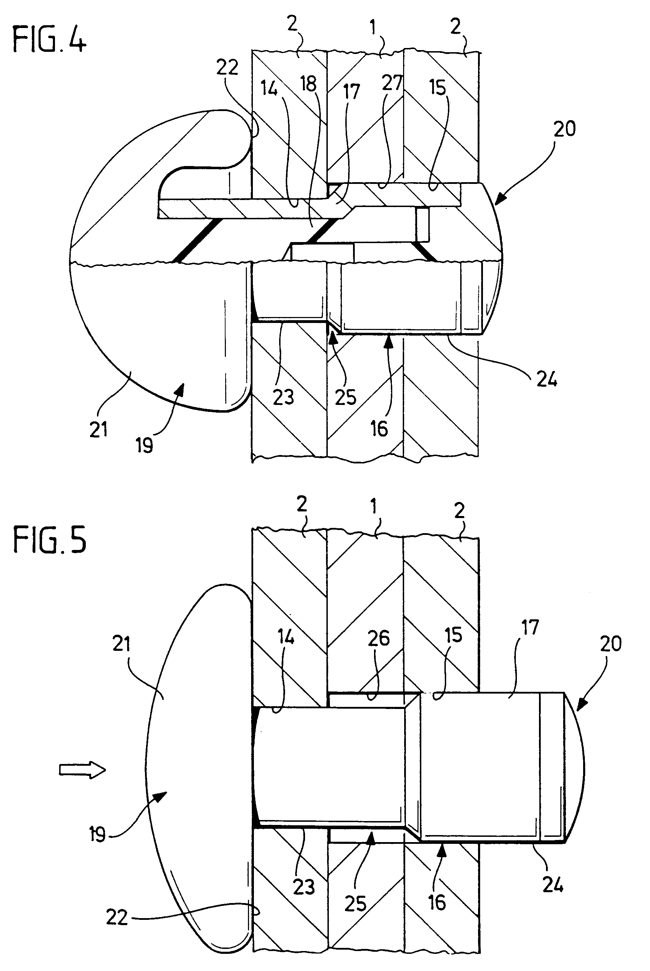

Die dem Kopf 21 naheliegende Öffnung 14 der Griffbranche 2 ist in ihren Abmessungen an einen Bereich 23 der Metallhülse 17 mit geringerem Außendurchmesser angepaßt, die weiter entfernte Öffnung 15 der Griffbranche 2 an einen Bereich 24 mit größerem Durchmesser. Die Lagerwelle 16 ist durch Druck auf den Kopf 21 so weit in die Öffnungen 14 und 15 einschiebbar, daß der Bereich 23 mit dem kleineren Durchmesser bis in die Öffnung 15 der Griffbranche 2 hineinreicht, also die gesamte Dicke der Griffbranche 1 durchsetzt, wie dies in Figur 5 dargestellt ist. Dabei verformt sich der Kopf 21 unter der Wirkung des Drucks auf die Lagerwelle 16 sehr stark. Beim Loslassen zieht der Kopf 21 daher aufgrund seiner elastischen Eigenschaften die Lagerwelle 16 wieder in die in Figur 4 dargestellte Ausgangslage zurück, wobei der Kopf 21 seine ursprüngliche Form wieder annimmt. Man erhält auf diese Weise eine rückstellende Federkraft, ohne daß dazu gesonderte Federelemente notwendig sind.

Die Griffbranche 1 weist im Bereich der Lagerstelle 5 eine Öffnung 25 auf, die sich in Längsrichtung der Griffbranche erstreckt und einen Mittelabschnitt 26 geringer Breite und an dessen beiden Enden je einen kreisförmigen Lagerabschnitt 27, 28 mit größerem Durchmesser umfaßt. Insgesamt ergibt sich damit eine Doppelschlüssellochform für diese Öffnung 25. Die Breite des Mittelabschnitts 26 ist dabei an den Durchmesser des Bereichs 23 der Lagerwelle angepaßt, der Durchmesser der Lagerabschnitte 27 und 28 an den Bereich 24 mit größerem Durchmesser.

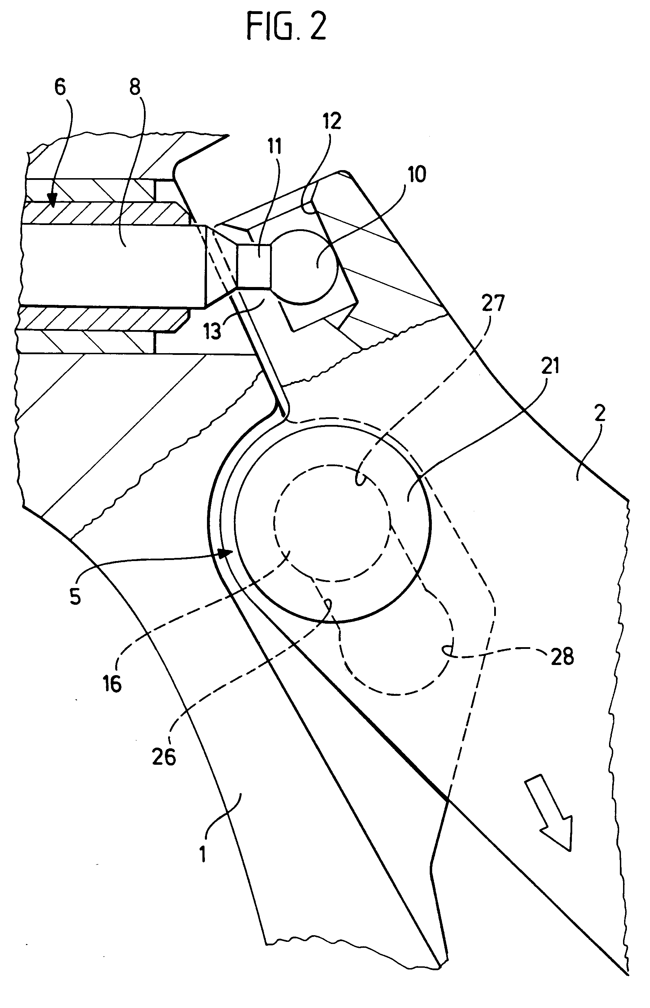

In dem normalen Lagerzustand, der in Figur 2 dargestellt ist, durchgreift die Lagerwelle 16 den Lagerabschnitt 27 der Griffbranche 1. Aufgrund der elastischen Rückstellkraft des Kopfs 21 wird die Lagerwelle 16 dabei so positioniert, daß ihr Bereich 24 in den Lagerabschnitt 27 eintaucht, die Griffbranche 1 ist damit an der Griffbranche 2 um eine feste Drehachse schwenkbar gelagert. Diese ist so angeordnet, daß in dieser Lagerposition das kugelige Ende 10 des Arbeitsteils 8 in die Sacklochbohrung 12 der Griffbranche 2 eintaucht (Figur 2).

Verschiebt man die Lagerwelle 16 in die in Figur 5 dargestellte, eingeschobene Position, so gelangt der Bereich 23 mit geringerem Durchmesser in die Öffnung 25, so daß jetzt die Griffbranche 1 gegenüber der Griffbranche 2 in Längsrichtung so lange verschoben werden kann, bis die Lagerwelle 16 in den gegenüberliegenden Lagerabschnitt 28 der Öffnung 25 eintaucht. Bei dieser Verschiebung tritt das kugelige Ende 10 des Arbeitsteils 8 aus der Sacklochbohrung 12 nach oben aus, so daß die Wirkverbindung zwischen Griffbranche 2 und Arbeitsteil 8 gelöst wird. Nach dem Eintritt der Lagerwelle 16 in der Lagerabschnitt 28 kann die Lagerwelle 16 wieder in ihre Ausgangslage verschoben werden, indem man den Kopf 21 losläßt. Aufgrund der elastischen Eigenschaften des Kopfs 21 wird dabei der Bereich 24 mit größerem Durchmesser in den Lagerabschnitt 28 eingeschoben, so daß nunmehr beide Griffbranchen wieder um eine definierte Achse miteinander verschwenkbar sind, diese ist jedoch gegenüber der ursprünglichen Lagerachse seitlich versetzt.

In gleicher Weise kann die Verschiebung in der umgekehrten Richtung erfolgen, wobei dabei das kugelige Ende 10 des Arbeitsteils 8 wieder in die Sacklochbohrung 12 eingeführt werden kann, so daß dann wieder eine Wirkverbindung zwischen der Griffbranche 2 und dem Arbeitsteil 8 hergestellt wird.

## Patentansprüche

1. Instrument für chirurgische Zwecke mit zwei relativ zueinander verschiebbaren Arbeitsteilen (6, 8) und mit zwei relativ zueinander verschwenkbaren Griffbranchen (1, 2), von denen eine mit dem einen und die andere mit dem anderen Arbeitsteil derart in Verbindung stehen, daß beim Schließen beziehungsweise Öffnen der Griffbranchen (1, 2) die beiden Arbeitsteile (6, 8) gegeneinander verschoben werden, wobei eine der beiden Griffbranchen (2) eine Lagerwelle (16) zur schwenkbaren Lagerung der anderen Griffbranche (1) trägt, die Bereiche (23, 24) mit unterschiedlichem Durchmesser aufweist und derart axial verschieblich ist, daß im Bereich der anderen Griffbranche (1) wahlweise ein Bereich (24) größeren oder ein Bereich (23) kleineren Durchmessers wirksam ist, wobei die andere Griffbranche (1) einen Schlitz (25) für die Lagerwelle (16) mit einem schmaleren Mittelabschnitt (26) und einem erweiterten Lagerabschnitt (27) an einem Ende aufweist, dessen Breite im schmaleren Mittelabschnitt (26) zwischen dem Durchmesser der beiden Bereiche (23, 24) der Lagerwelle (16) liegt und in dem kreisförmig erweiterten Lagerabschnitt (27) einen Durchmesser aufweist, der dem größeren Durchmesser (Bereich 24) der Lagerwelle (16) entspricht, und wobei eine der beiden Griffbranchen (2) mit einem Mitnehmer (12) gelenkig an dem einen Arbeitsteil (8) angreift, wenn die Lagerwelle (16) den erweiterten Lagerabschnitt (27) durchsetzt, dadurch gekennzeichnet, daß am anderen Ende des Mittelabschnitts (26) ein anderer erweiterter Lagerabschnitt (28) mit einem ebenso erweiterten Durchmesser angeordnet ist und daß der Mitnehmer (12) das Arbeitsteil (8) freigibt, wenn die Lagerwelle (16) diesen anderen erweiterten Lagerabschnitt (28) durchsetzt.

2. Instrument nach Anspruch 1, dadurch gekennzeichnet, daß die Lagerwelle (16) unter Federbelastung in eine Position verschoben wird, in der der Bereich (24) mit größerem Durchmesser an der auf der Lagerwelle (16) gelagerten Griffbranche wirksam ist.

3. Instrument nach Anspruch 2, dadurch gekennzeichnet, daß die Lagerwelle (16) einen sich an der Außenseite einer Griffbranche abstützenden Kopf (21) aus elastisch verformbarem Material aufweist, der sich unter dauerhafter Abstützung an der Griffbranche über den gesamten axialen Verschiebeweg der Lagerwelle (16) elastisch verformen läßt.

4. Instrument nach Anspruch 3, dadurch gekennzeichnet, daß der Kopf (21) im unverformten Zustand die Form einer etwa kugeligen Halbschale aufweist, die sich mit ihrem Rand (22) an der Griffbranche abstützt und die in ihrem zentralen Teil mit der Lagerwelle (16) verbunden ist.

5. Instrument nach Anspruch 3 oder 4, dadurch gekennzeichnet, daß die Lagerwelle (16) eine stufige Metallhülse (17) umfaßt, in die ein mit dem Kopf (21) verbundener Schaft (18) eingesteckt ist.

6. Instrument nach Anspruch 5, dadurch gekennzeichnet, daß von der gegenüberliegenden Seite des Schafts (18) ein die Metallhülse (17) stirnseitig überfangendes Gegenstück (20) in diese eingeführt ist, welches mit dem Schaft (18) verbunden ist.

7. Instrument nach Anspruch 6, dadurch gekennzeichnet, daß die Verbindung zwischen Schaft (18) und Gegenstück (20) eine Rastverbindung ist.

8. Instrument nach einem der voranstehenden Ansprüche, dadurch gekennzeichnet, daß das bewegte Arbeitsteil (8) ein kugeliges Ende (10) aufweist, das über einen Steg (11) mit kleineren Abmessungen mit dem Arbeitsteil (8) in Verbindung steht.

9. Instrument nach Anspruch 8, dadurch gekennzeichnet, daß der Mitnehmer (12) eine im wesentlichen parallel zur schlitzförmigen Öffnung (25) verlaufende Sacklochbohrung in der Griffbranche (2) ist, die über einen seitlichen Längsschlitz (13) offen ist, dessen Breite kleiner ist als der Durchmesser des kugeligen Endes (10) und größer als die Breite des Stegs (11).

10. Instrument nach einem der voranstehenden Ansprüche, dadurch gekennzeichnet, daß der Schlitz (25) im wesentlichen senkrecht zur Verschieberichtung des Arbeitsteils (8) angeordnet ist.

## Claims

1. An instrument for surgical use, comprising two working parts (6, 8) displaceable relative to one another and two gripping arms (1, 2) pivotable relative to one another, one of which is connected to one of the working parts and the other being connected to the other working part in such a manner that the two working parts (6, 8) are mutually displaced when the gripping arms (1, 2) are opened or closed, wherein one of the two gripping arms (2) carries a spindle (16) for pivotably mounting the other gripping arm (1), the spindle (16) having regions (23, 24) of different diameters and being axially displaceable in such a manner that, in the region of the other gripping arm (1), either a region (24) of larger diameter or a region (23) of smaller diameter is operative, wherein the other gripping arm (1) has a slot (25) for the spindle (16) with a narrower central portion (26) and a widened mounting portion (27) at one end, the width of which in the narrower central portion (26) lies between the diameter of the two regions (23, 24) of the spindle (16) and, in the widened mounting portion (27), widened in the shape of a circle, has a diameter corresponding to the larger diameter (region 24) of the spindle (16), and wherein one of the two gripping arms (2) engages in an articulated manner on the one working part (8) by means of a carrier (12) when the spindle (16) extends through the widened mounting portion (27), characterised in that another widened mounting portion (28) of equally enlarged diameter is arranged at the other end of the central portion (26) and in that the carrier (12) releases the working part (8) when the spindle (16) extends through the other widened mounting portion (28).

2. An instrument according to claim 1, characterised in that the spindle (16) is displaced under spring load into a position in which the region (24) of larger diameter is operative on the gripping arm mounted on the spindle (16).

3. An instrument according to claim 2, characterised in that the spindle (16) has a head (21) supported against the outside of one gripping arm and comprising elastically deformable material which is elastically deformable over the entire axial displacement path of the spindle (16) while being constantly supported against the gripping arm.

4. An instrument according to claim 3, characterised in that, in the non-deformed state, the head (21) is in the shape of a substantially spherical hemisphere, the edge (22) of which is supported against the gripping arm and the central part of which is connected to the spindle (16).

5. An instrument according to claim 3 or 4, characterised in that the spindle (16) comprises a stepped metal sleeve (17), into which is inserted a shaft (18) connected to the head (21).

6. An instrument according to claim 5, characterised in that a counterpart (20), covering the end of the metal sleeve (17) and connected to the shaft (18), is inserted into the metal sleeve (17) from the opposing end of the shaft (18).

7. An instrument according to claim 6, characterised in that the connection between the shaft (18) and the counterpart (20) is a snap-locking connection.

8. An instrument according to any one of the preceding claims, characterised in that the movable working part (8) has a spherical end (10) connected thereto by a stem (11) of smaller dimensions.

9. An instrument according to claim 8, characterised in that the carrier (12) is a blind hole provided in the gripping arm (2) and extending substantially parallel to the slot-type opening (25), the blind hole being open along a lateral longitudinal slot (13) of a width smaller than the diameter of the spherical end (10) and larger than the width of the stem (11).

10. An instrument according to any one of the preceding claims, characterised in that the slot (25) is arranged substantially perpendicularly to the direction of displacement of the working part (8).

## Revendications

1. Instrument pour utilisation chirurgicale, comprenant deux parties fonctionnelles (6, 8) capables de se déplacer l'une par rapport l'autre, et comprenant deux branches de saisie (1, 2) capables de basculer l'une par rapport à l'autre, dont l'une est reliée à l'une des parties fonctionnelles, et l'autre est reliée à l'autre partie fonctionnelle, de telle manière que lors de la fermeture ou respectivement de l'ouverture des branches de saisie (1, 2) les deux parties fonctionnelles (6, 8) sont déplacées l'une par rapport à l'autre, l'une des deux branches de saisie (2) portant un arbre de montage (16) pour le montage en basculement de l'autre branche de saisie (1), ledit arbre comportant des zones (23, 24) de diamètres différents, et étant mobile axialement, de manière que dans la région de l'autre branche de saisie (1) est active au choix une zone (24) de grand diamètre ou une zone (23) de petit diamètre, l'autre branche de saisie (1) comportant une fente (25) pour l'arbre de montage (16) avec un tronçon médian étroit (26) et un tronçon de montage élargi (27) à une extrémité, dont la largeur dans le tronçon médian étroit (26) est située entre les diamètres des deux zones (23, 24) de l'arbre de montage (16), et présente dans le tronçon de montage élargi de forme circulaire (27) un diamètre qui correspond au grand diamètre (zone 24) de l'arbre de montage (16), et l'une des deux branches de saisie (2) attaque au moyen d'un élément d'entraînement (12) de manière articulée l'une des parties fonctionnelles (8) lorsque l'arbre de montage (16) traverse le tronçon de montage élargi (27), caractérisé en ce que, à l'autre extrémité du tronçon médian (26) est agencé un autre tronçon de montage élargi (28) avec un diamètre également élargi, et en ce que l'élément d'entraînement (12) libère la partie fonctionnelle (8) lorsque l'arbre de montage (16) traverse cet autre tronçon de montage élargi (28).

2. Instrument selon la revendication 1, caractérisé en ce que l'arbre de montage (16) est déplacé sous une charge élastique jusque dans une position dans laquelle la zone (24) de grand diamètre agit sur la branche de saisie montée sur l'arbre de montage (16).

3. Instrument selon la revendication 2, caractérisé en ce que l'arbre de montage (16) comprend une tête (21) en matériau élastiquement déformable, qui s'appuie contre la face extérieure d'une branche de saisie, ladite tête pouvant être déformée de manière élastique sous un appui durable contre la branche de saisie sur la totalité du trajet de déplacement axial de l'arbre de montage (16).

4. Instrument selon la revendication 3, caractérisé en ce que la tête (21) présente dans la l'état non déformé approximativement la forme d'une demi-coque sphérique, qui s'appuie par sa bordure (22) contre la branche de saisie, et qui est reliée dans sa partie centrale à l'arbre de montage (16).

5. Instrument selon l'une ou l'autre des revendications 3 et 4, caractérisé en ce que l'arbre de montage (16) comprend une douille métallique (17) en gradins, dans laquelle est enfilée une tige (18) reliée à la tête (21).

6. Instrument selon la revendication 5, caractérisé en ce que, depuis le côté opposé de la tige (18), une pièce complémentaire (20) qui coiffe du côté frontal la douille métallique (17) est enfilée dans celle-ci, ladite pièce complémentaire étant reliée avec la tige (18).

7. Instrument selon la revendication 6, caractérisé en ce que la liaison entre la tige (18) et la pièce complémentaire (20) est une liaison à enclenchement.

8. Instrument selon l'une des revendications précédentes, caractérisé en ce que la partie fonctionnelle mobile (8) comporte une extrémité sphérique (10), qui est reliée à la partie fonctionnelle (8) au moyen d'une barrette (11) de plus petites dimensions.

9. Instrument selon la revendication 8, caractérisé en ce que l'élément d'entraînement (12) est un perçage borgne dans la branche de saisie (2) qui s'étend sensiblement parallèlement à l'ouverture en forme de fente (25), ledit perçage borgne étant ouvert au moyen d'une fente longitudinale latérale (13) dont la largeur est inférieure au diamètre de l'extrémité sphérique (10) et supérieure à la largeur de la barrette (11).

10. Instrument selon l'une des revendications précédentes, caractérisé en ce que la fente (25) est agencée sensiblement perpendiculairement à la direction de déplacement de la partie fonctionnelle (8).
